# EUROPEAN PATENT APPLICATION

(11) **EP 4 772 141 A1**
(43) Date of publication of application: **08.07.2026**
(21) Application number: 24857590.4
(22) Date of filing: 10.01.2024
(51) Int. Cl.: A61D 1/00, A61B 17/3211, A61B 17/32

(54) **URETHROTOME FOR INTERNAL URETHROTOMY FOR PET**

(30) Priority: 31.08.2023 CN 202311115069; 13.10.2023 CN 202311325205
(71) Applicant: Li, Li, Chuzhou, Anhui 239499 (CN)
(72) Inventor: LI, Li, Chuzhou, Anhui 239499 (CN); ZHAO, Guowen, Chuzhou, Anhui 239499 (CN)
(74) Representative: Metida
(86) International application number: PCT/CN2024/071589
(87) International publication number: WO 2025/044032

(57) **Abstract**

Disclosed in the present invention is an urethrotome for internal urethrotomy for a pet. The urethrotome includes an urethrotome rod, a blade, and a guide rod; a blade slot is formed in one side of the urethrotome rod, and a hollow structure is arranged in the urethrotome rod in a lengthwise direction and is in communication with the bottom of the blade slot; the blade is rotatably accommodated in the blade slot; and one end of the guide rod abuts against an edge of one end of the blade, and the other end of the guide rod passes through the hollow structure and forms an operation end at one end of the urethrotome rod, such that one end of the guide rod is driven by the operation end to push the blade to rotate and protrude from an opening of the blade slot. In this way, the blade can be hidden and stored in the blade slot during the process of inserting the urethrotome into the urethra of the pet, such that the urethrotome rod can be inserted into the urethra of the pet more smoothly; and if the blade reaches a strictured and obstructed part of the urethra of the pet, the guide rod is driven by means of the operation end to push the blade to protrude from the opening of the blade slot and perform surgical cutting on the strictured and obstructed part of the urethra of the pet. The urethrotome has a simple and flexible structural design, and is more convenient to operate.

## Description

### TECHNICAL FIELD

The present invention relates to the technical field of medical instruments for pets, and particularly relates to an urethrotome for internal urethrotomy for a pet.

### BACKGROUND

Currently, in the field of veterinary clinical medicine, urodialysis caused by urethral stricture is one of the common diseases in urology, especially in male cats with relatively strictured urethra. Once the male cat develops urodialysis, it could die within a few days, and the mortality rate is very high. The main causes of urodialysis in the pets are: (1) stones or crystals blocking the urethra or ureters; (2) spontaneous cystitis; (3) urinary tract infections; (4) stress-induced urodialysis, etc. The conventional treatment for urodialysis in the pets involves urethrostomy or oral medication. However, conventional urethrostomy often has many sequelae, and oral medication takes a long time to take effect.

In existing technology, there are also treatment methods that use an urethrotome for surgical cutting, for example, Chinese invention patent with application number of CN201520758495.4 discloses an urethrotome for internal urethrotomy, the urethrotome for internal urethrotomy includes a cylindrical hollow urethrotome rod, a guide rod passes through the urethrotome rod all the time, one side of the urethrotome rod is equipped with a curved blade, the urethrotome rod and the blade form an integrated structure, and the blade protrudes from one side of the urethrotome rod. Due to the outward protruding fixation of the blade relative to the urethrotome rod, the overall width of the urethrotome for internal urethrotomy is inadvertently increased, requiring a doctor to be especially careful during inserting the urethrotome for internal urethrotomy into the strictured urethra of the pet to prevent cutting the normal parts of the urethra of the pet. Moreover, the doctor must slow down the speed in the surgical operation, otherwise, inserting the rigid urethrotome rod and blade into the strictured urethra of the pet could easily cause urethral scratches, making the surgical cutting more difficult and inconvenient to use.

### SUMMARY

An objective of the present invention is to provide an urethrotome for internal urethrotomy for a pet, and to solve the technical problems that the urethrotome for internal urethrotomy for the pet in existing technology is difficult in operation, and inconvenient to use.

In order to achieve above objective, the technical solution of the present invention provides the urethrotome for internal urethrotomy for the pet, and the urethrotome for internal urethrotomy for the pet includes:
an urethrotome rod, a blade slot being formed in one side of the urethrotome rod, and a hollow structure being arranged in the urethrotome rod in a lengthwise direction and being in communication with the bottom of the blade slot;
a blade which is rotatably accommodated in the blade slot and has an accommodated state and a protruded state relative to the blade slot; and
a guide rod of which one end abuts against an edge of one end of the blade while the other end passes through the hollow structure and forms an operation end at one end of the urethrotome rod, one end of the guide rod being driven by the operation end to push the blade to rotate and protrude from an opening of the blade slot, so as to switch the blade from the accommodated state to the protruded state.

In one possible implementation, a slope is arranged on the edge of one end of the blade; and in the protruded state, the blade abuts against one end of the guide rod through the slope so as to be supported for positioning.

In one possible implementation, in the accommodated state, the blade is accommodated in the blade slot, the lengthwise position of the blade is parallel to a longitudinal axis of the urethrotome rod, and the slope is inclined relative to the longitudinal axis of the urethrotome rod; and in the protruded state, the other end of the blade protrudes from the opening of the blade slot, the lengthwise position of the blade is inclined relative to the longitudinal axis of the urethrotome rod, and the slope is parallel to the longitudinal axis of the urethrotome rod.

In one possible implementation, the blade slot extends in the lengthwise position of the urethrotome rod, one end of the blade is rotatably mounted in the blade slot through a pivot, and the pivot extends in a width direction of the blade slot.

In one possible implementation, further comprising a mounting body, wherein a mounting groove is formed in one side of the mounting body and corresponds to the blade slot, mounting holes are formed in two opposite side walls of the mounting groove, the blade is inserted into the mounting groove, and two ends of the pivot are inserted into the two mounting holes respectively so as to realize rotatable mounting the blade; and the mounting body is inserted into the hollow structure of the urethrotome rod so as to rotatably mount the blade in the blade slot.

In one possible implementation, a penetrating hole is formed in the mounting body in the lengthwise direction and is in communication with the bottom of the mounting groove; one end of the guide rod is movably inserted into the penetrating hole; in the accommodated state, the blade at least partially passes through the bottom of the mounting groove and abuts against the guide rod in the penetrating hole, and then the blade can be pushed by the guide rod to rotate and protrude from the opening of the blade slot.

In one possible implementation, further comprising a pushing member, wherein the pushing member is movably arranged in the hollow structure of the urethrotome rod, and one end of the guide rod is connected to the pushing member and then abuts against the edge of one end of the blade by the pushing member; and the guide rod can be driven by the operation end to drive the pushing member to push the blade to rotate and protrude from the opening of the blade slot.

In one possible implementation, the blade slot extends in the lengthwise position of the urethrotome rod, the other end of the blade is rotatably mounted in the blade slot through the pivot, and a pivotal axis of the pivot extends in the width direction of the blade slot.

In one possible implementation, further comprising the mounting body, wherein the mounting groove is formed in the side, facing the opening of the blade slot, of the mounting body in the lengthwise position, the other end of the blade is rotatably inserted in the mounting groove through the pivot, and the mounting body is inserted in the hollow structure of the urethrotome rod so as to rotatably mount the blade in the blade slot.

In one possible implementation, a guide surface is arranged on the edge of one end of the blade; a guide groove is formed in the end, close to the blade, of the pushing member; one end of the blade is movably arranged in the guide groove through the guide surface; and the pushing member abuts against the guide surface through the guide groove to push the blade to rotate and protrude from the opening of the blade slot.

In one possible implementation, further comprising the mounting body, wherein the mounting groove is formed in the side, facing the opening of the blade slot, of the mounting body in the lengthwise position; the other end of the blade is rotatably arranged in the mounting groove by the pivot; the penetrating hole is formed in the mounting body in the lengthwise direction and is in communication with the bottom of the mounting groove; the pushing member is movably arranged in the penetrating hole; one end of the guide rod is inserted into the penetrating hole and connected to the pushing member, and the mounting body is inserted into the hollow structure of the urethrotome rod so as to rotatably mount the blade in the blade slot; and in the accommodated state, the blade at least partially passes through the bottom of the mounting groove and abuts against the pushing member in the penetrating hole, and then the guide rod can be driven by the pushing member to push the blade to rotate and protrude from the opening of the blade slot.

In one possible implementation, the urethrotome rod is of a flexible rod structure, or the urethrotome rod consists of a flexible rod portion and a hard rod portion.

In one possible implementation, further comprising a handle and a driving member, wherein the handle is arranged at one end of the urethrotome rod, the driving member is movably arranged on the handle and is in driving connection with the operation end of the guide rod, and the guide rod is driven by the driving member to push the blade to protrude from the opening of the blade slot.

In one possible implementation, the driving member comprises a rotating member, the rotating member is in threaded connection with the handle and is connected to the operation end of the guide rod, and the rotating member can be rotated to drive the guide rod to push the blade to rotate and protrude from the opening of the blade slot; or
the driving member comprises a slider which is connected to the operation end of the guide rod, at least one side of the slider is provided with a sliding portion, the handle is provided with a leading channel in the lengthwise position, and the sliding portion is arranged in the leading channel in a sliding mode; and the slider can be slid to drive the guide rod to push the blade to rotate and protrude from the opening of the blade slot.

In one possible implementation, further comprising the handle and the driving member, wherein the handle is arranged at one end of the urethrotome rod; the driving member comprises the rotating member, the rotating member is in threaded connection with the handle and is connected to the operation end of the guide rod; the pushing member is provided with a threaded hole in the lengthwise position, and one end of the guide rod is in threaded connection with the threaded hole; the pushing member is inserted into the hollow structure of the urethrotome rod in a non-rotatable mode, and the rotating member can be rotated to drive the guide rod to move the pushing member; or
further comprising the handle and the driving member, wherein the handle is arranged at one end of the urethrotome rod; the driving member comprises the slider which is connected to the operation end of the guide rod; the sliding portion is arranged on at least one side of the slider; the handle is provided with the leading channel in the lengthwise position, and the sliding portion is arranged in the leading channel in a sliding mode; the slider can be slid to drive the guide rod to move the pushing member; or
further comprising the handle and the driving member, wherein the handle is arranged at one end of the urethrotome rod; the driving member comprises the rotating member and the slider; the slider is connected to the operation end of the guide rod, and the sliding portion is arranged on at least one side of the slider; the handle is provided with the leading channel in the lengthwise position, and the sliding portion is arranged in the leading channel in a sliding mode; the rotating member is in threaded connection with the handle and abuts against the slider; and the rotating member can be rotated to push the slider to slide so as to drive the guide rod to move the pushing member.

According to the above technical solution, the urethrotome for internal urethrotomy for the pet provided by the present invention includes the urethrotome rod, the blade, and the guide rod; the blade slot is formed in one side of the urethrotome rod, and the hollow structure is arranged in the urethrotome rod in the lengthwise direction and is in communication with the bottom of the blade slot; the blade is rotatably accommodated in the blade slot and has the accommodated state and the protruded state relative to the blade slot; one end of the guide rod abuts against the edge of one end of the blade, and the other end of the guide rod passes through the hollow structure and forms the operation end at one end of the urethrotome rod, such that one end of the guide rod is driven by the operation end to push the blade to rotate and protrude from the opening of the blade slot, so as to switch the blade from the accommodated state to the protruded state; in this way, the blade can be hidden and stored in the blade slot during inserting the urethrotome inserted into the urethra of the pet to prevent cutting the normal parts of the urethra of the pet, such that the urethrotome rod can be inserted into the urethra of the pet more smoothly; and if the blade reaches a strictured and obstructed part of the urethra of the pet, the guide rod is driven by means of the operation end to push the blade to protrude from the opening of the blade slot and perform surgical cutting on the strictured and obstructed part of the urethra of the pet. The urethrotome has a simple and flexible structural design, and is more convenient to operate, and the surgical operation difficulty is effectively reduced.

In order to make the technical concept and other objectives, advantages, features, and functions of the present invention clearer and easier to understand, preferred embodiments will be given in the following specific implementation methods, and detailed explanations will be made in conjunction with the accompanying drawings.

### BRIEF DESCRIPTION OF THE DRAWINGS

In order to more clearly illustrate the technical solutions in the embodiments of the present invention, the accompanying drawings in the description of the embodiments will be briefly introduced below. Obviously, the accompanying drawings described below are only some embodiments of the present invention. For those of ordinary skill in the art, it is possible to obtain other drawings from these accompanying drawings without any creative work.
FIG. 1 is a schematic structural diagram of an urethrotome in an accommodated state according to one embodiment of the present invention;
FIG. 2 is a schematic structural diagram of an urethrotome in a protruded state according to one embodiment of the present invention;
FIG. 3 is a simplified schematic diagram of an urethrotome in an accommodated state according to one embodiment of the present invention;

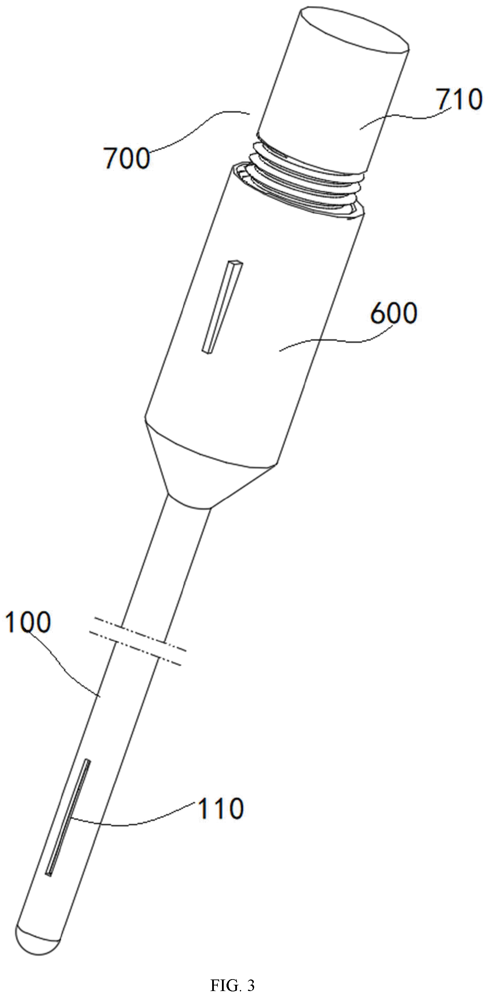

FIG. 3 is a simplified schematic diagram of an urethrotome in a protruded state according to one embodiment of the present invention;

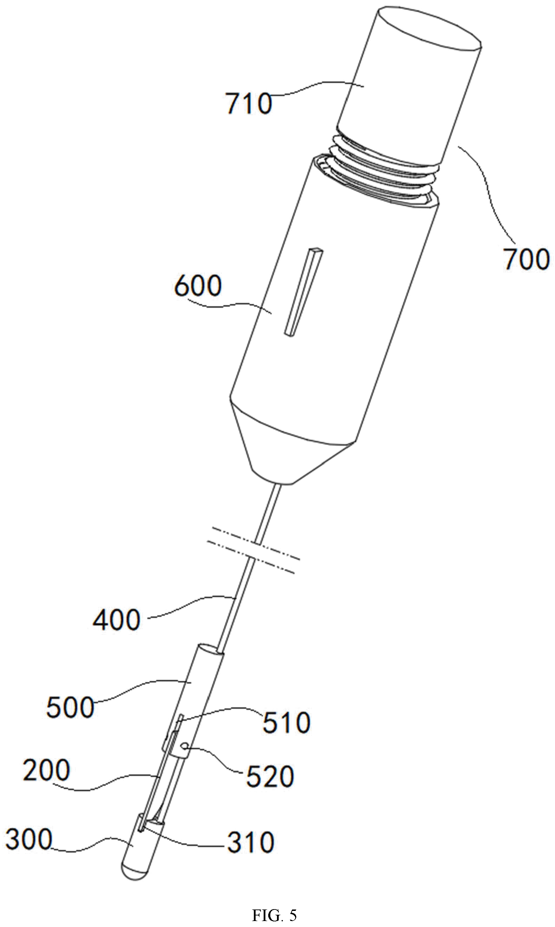

FIG. 5 is a simplified schematic diagram without an urethrotome rod in FIG. 3 according to one embodiment of the present invention;

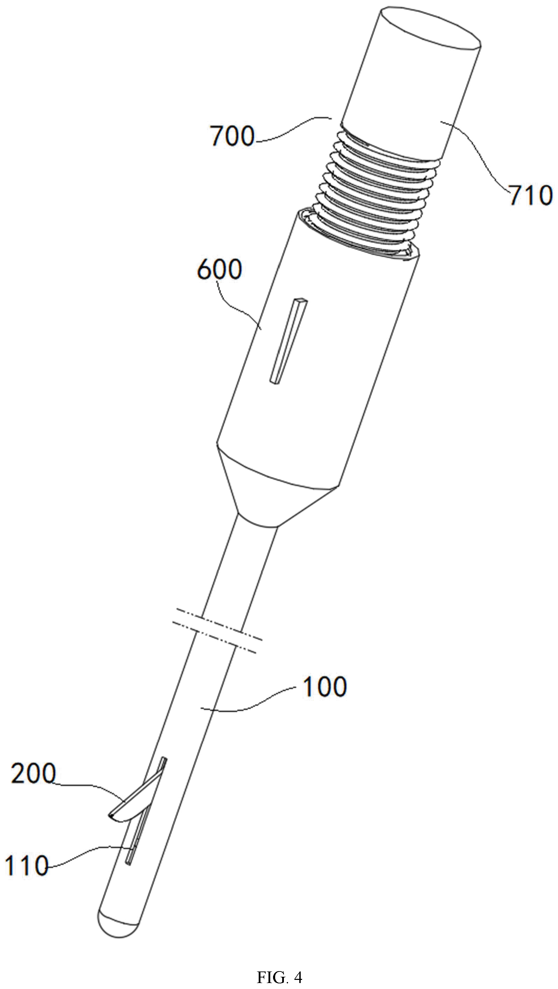

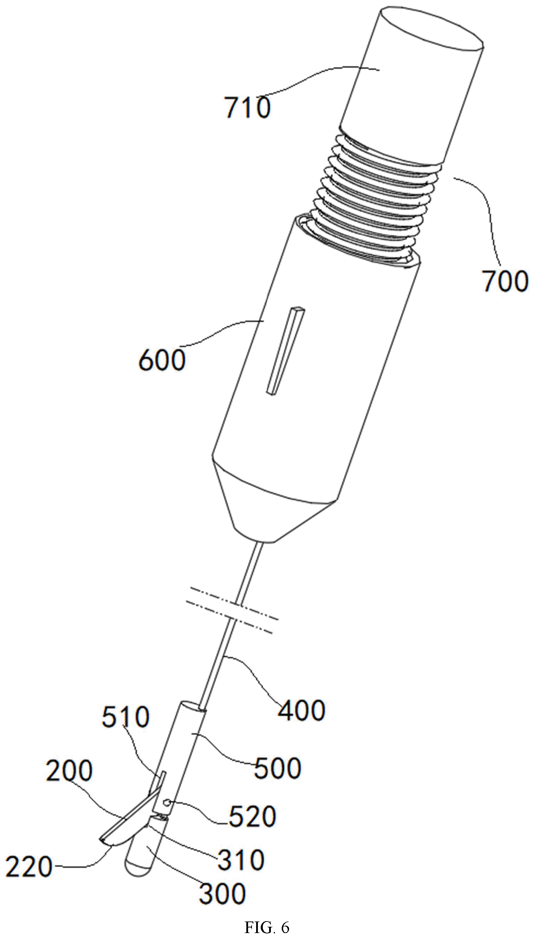

FIG. 6 is a simplified schematic diagram without an urethrotome rod in FIG. 4 according to one embodiment of the present invention;

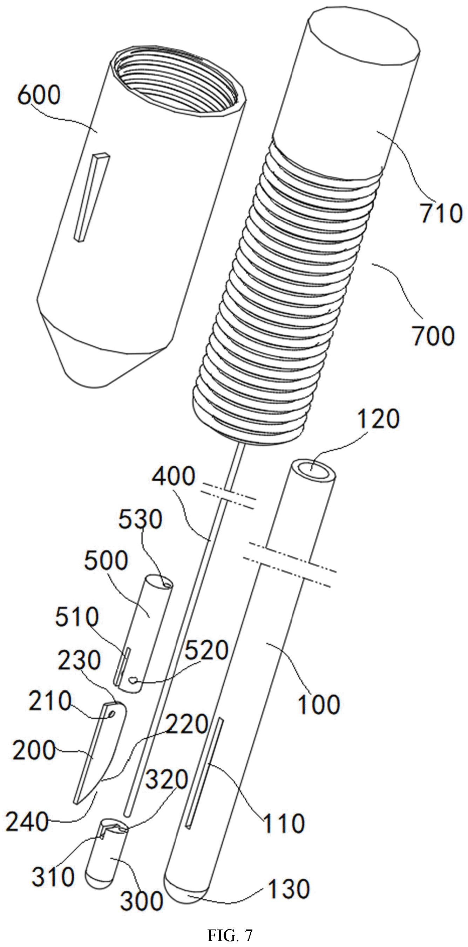

FIG. 7 is an exploded view of an urethrotome according to one embodiment of the present invention;

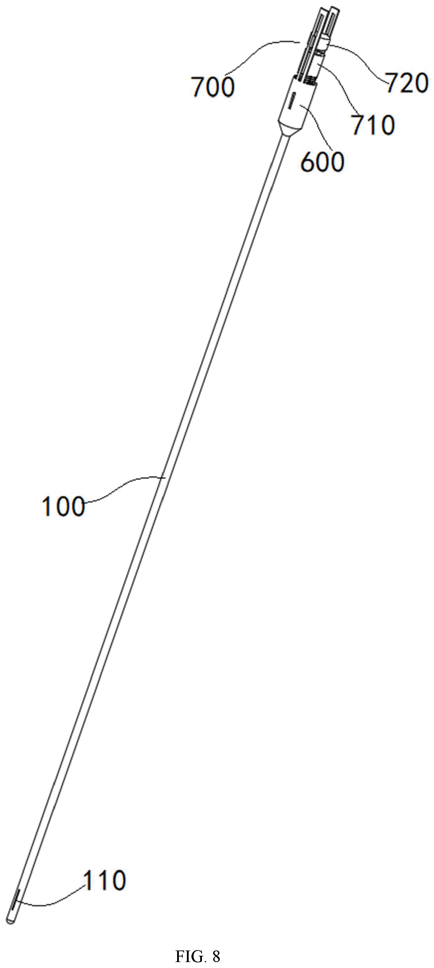

FIG. 8 is a schematic structural diagram of an urethrotome in an accommodated state according to another embodiment of the present invention;

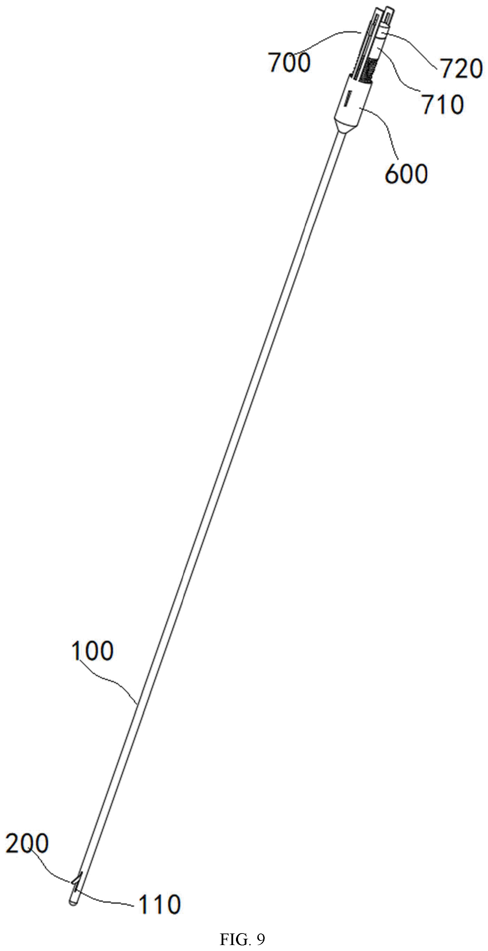

FIG. 9 is a schematic structural diagram of an urethrotome in a protruded state according to another embodiment of the present invention;

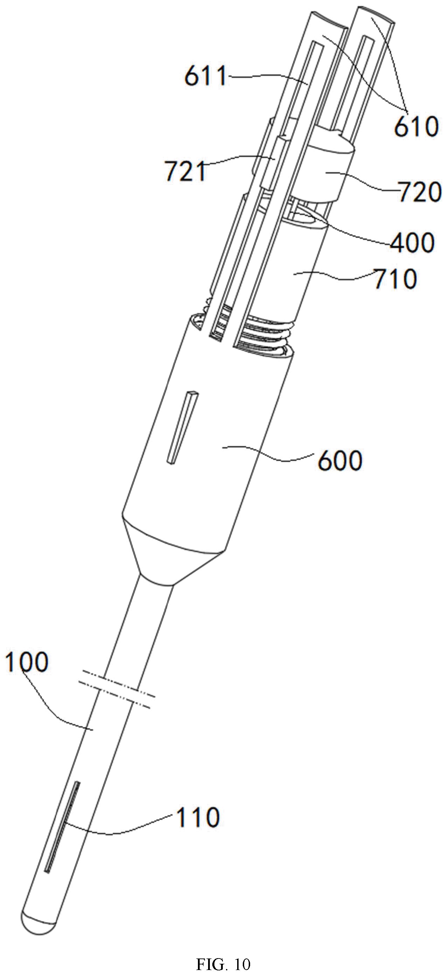

FIG. 10 is a simplified schematic diagram of an urethrotome in an accommodated state according to another embodiment of the present invention;

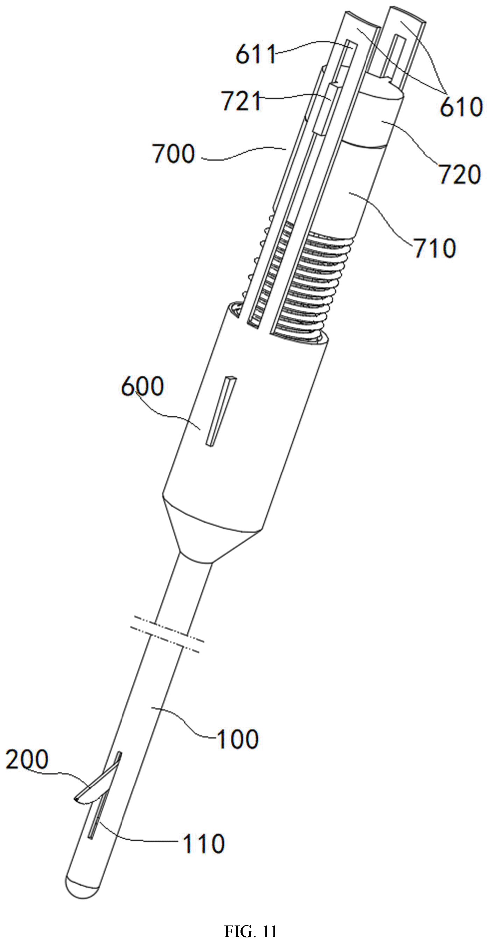

FIG. 11 is a simplified schematic diagram of an urethrotome in a protruded state according to another embodiment of the present invention;

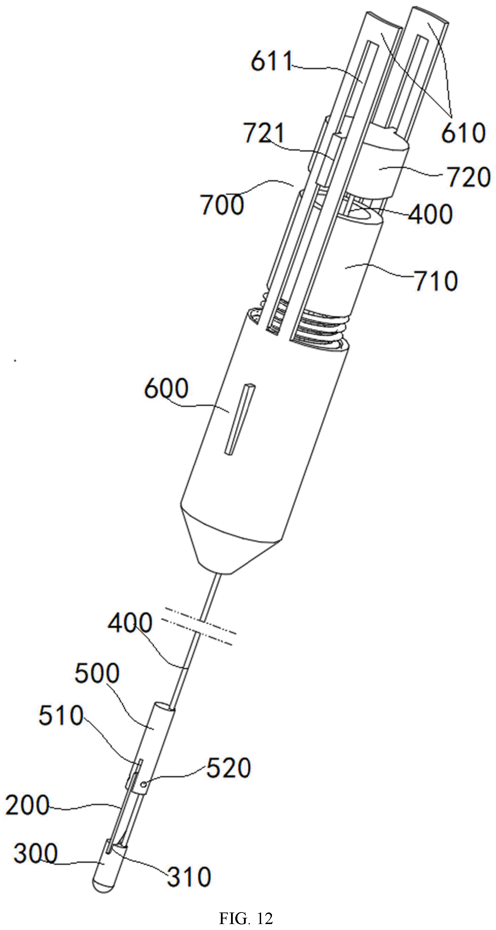

FIG. 12 is a simplified schematic diagram without an urethrotome rod in FIG. 10 according to one embodiment of the present invention;

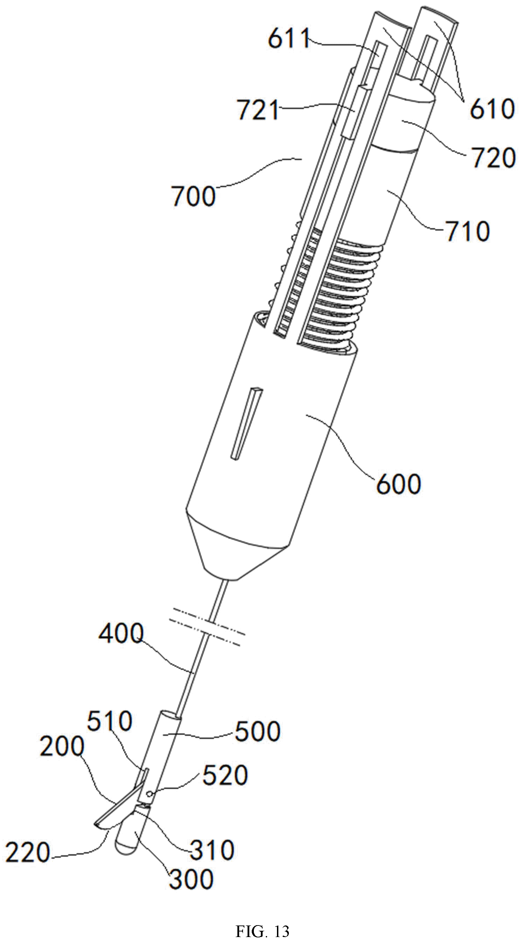

FIG. 13 is a simplified schematic diagram without an urethrotome rod in FIG. 11 according to one embodiment of the present invention;

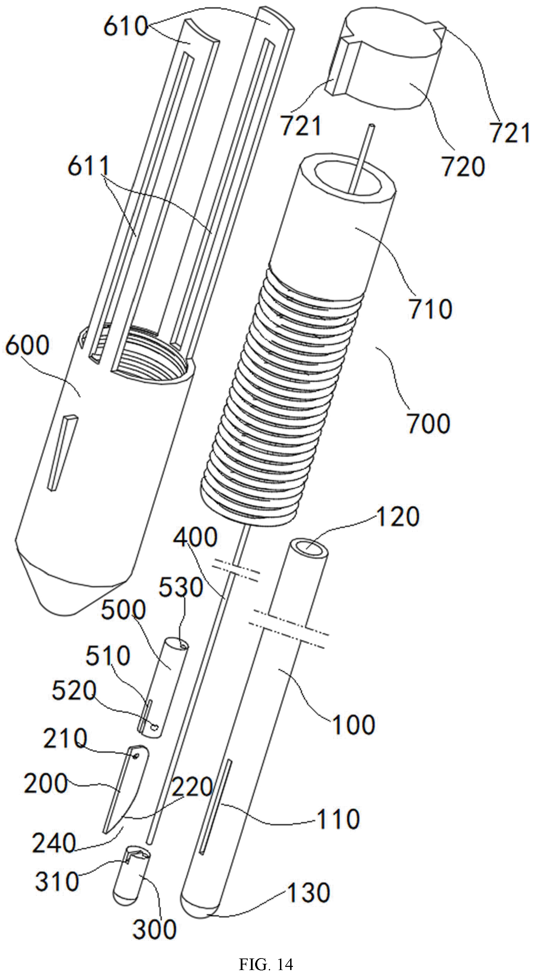

FIG. 14 is an exploded view of an urethrotome according to another embodiment of the present invention;

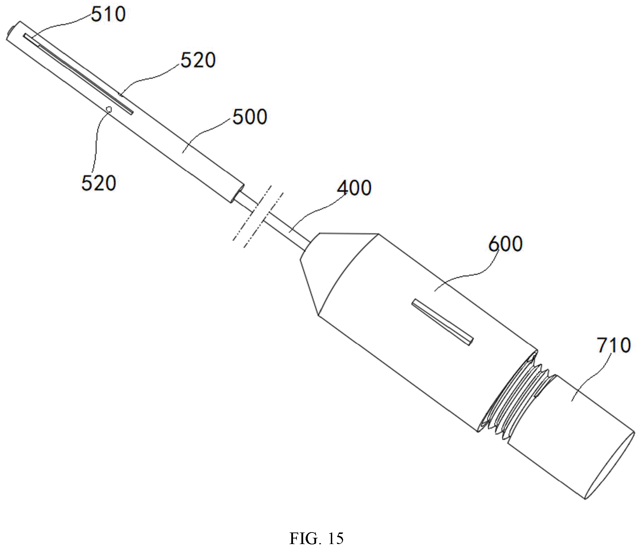

FIG. 15 is a simplified schematic diagram of an urethrotome in an accommodated state according to yet another embodiment of the present invention;

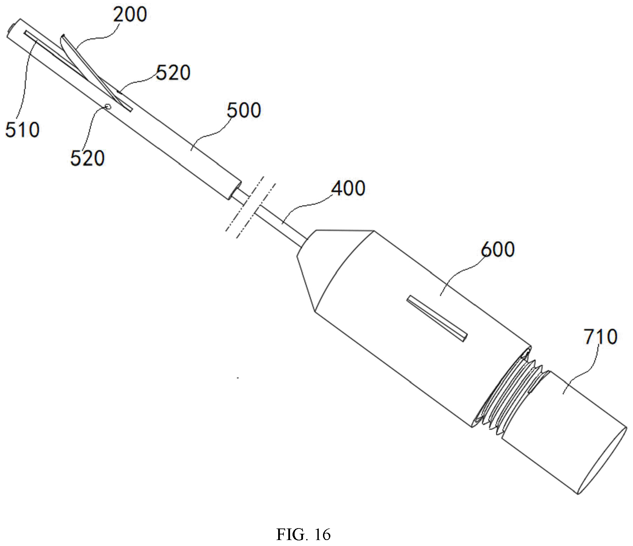

FIG. 16 is a simplified schematic diagram of an urethrotome in a protruded state according to yet another embodiment of the present invention; and

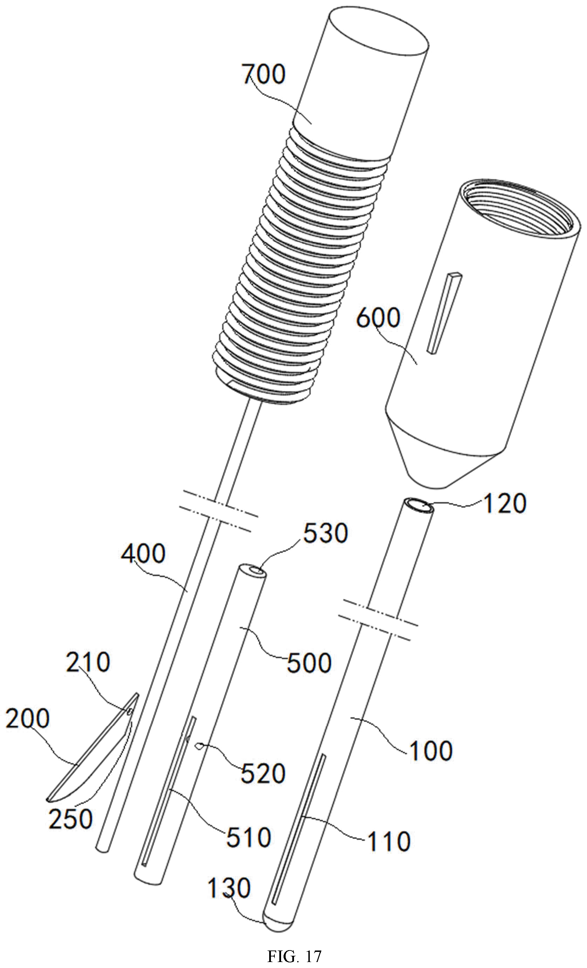

FIG. 17 is an exploded view of an urethrotome according to yet another embodiment of the present invention.

### Reference numerals in the figures:

100, urethrotome rod; 110, blade slot; 120, hollow structure; 130, end cap;
200, blade; 210, through hole; 220, guide surface; 230, first end; 240, second end; 250, slope;
300, pushing member; 310, guide groove; 320, threaded hole;
400, guide rod; 500, mounting body; 510, mounting groove; 520, mounting hole; 530, penetrating hole;
600, handle; 610, guide wall; 611, leading channel;
700, driving member; 710, rotating member; 720, slider; and 721, sliding portion.

### DETAILED DESCRIPTION

An embodiment of the present invention provides an urethrotome for internal urethrotomy for a pet, aiming to solve the technical problems that the urethrotome for internal urethrotomy for the pet in existing technology is difficult in operation, and inconvenient to use; a blade slot 110 is formed in one side of an urethrotome rod 100, such that the blade can be hidden and stored in the blade slot 110 during inserting the urethrotome into the urethra; in this way, the urethrotome rod 100 and a blade 200 can be inserted into the urethra of the pet more smoothly; if the blade 200 reaches a strictured and obstructed part of the urethra of the pet, a guide rod 400 is driven by means of an operation end to drive a pushing member 300 to push the blade 200 to protrude from an opening of the blade slot 110 and perform surgical cutting on the strictured and obstructed part of the urethra of the pet; and the urethrotome is convenient to use and easy to operate.

In order to enable those of skill in the art to better understand the technical solutions of the present invention, the technical solutions in the embodiments of the present invention will be clearly and completely described in conjunction with the accompanying drawings of the embodiments of the present invention below. Obviously, the described embodiments are merely part of the embodiments of the present invention and not all of them. Based on the embodiments of the present invention, all other embodiments obtained by those skilled in the art without inventive efforts also fall within the scope of protection of the present invention.

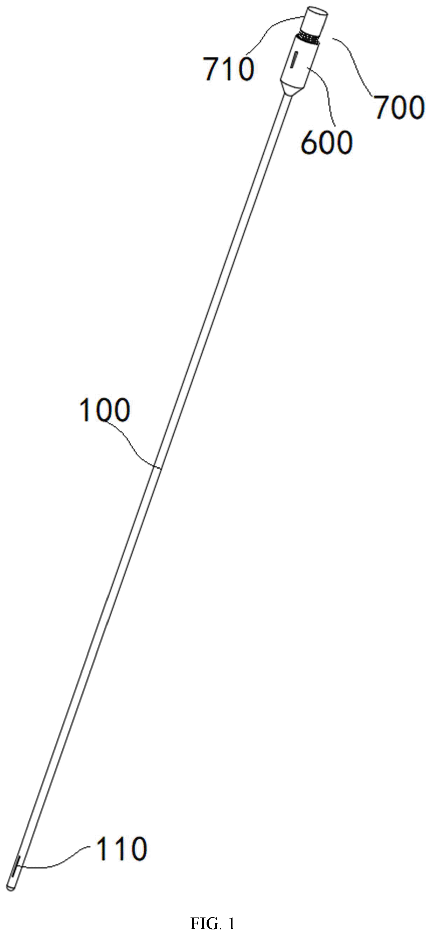

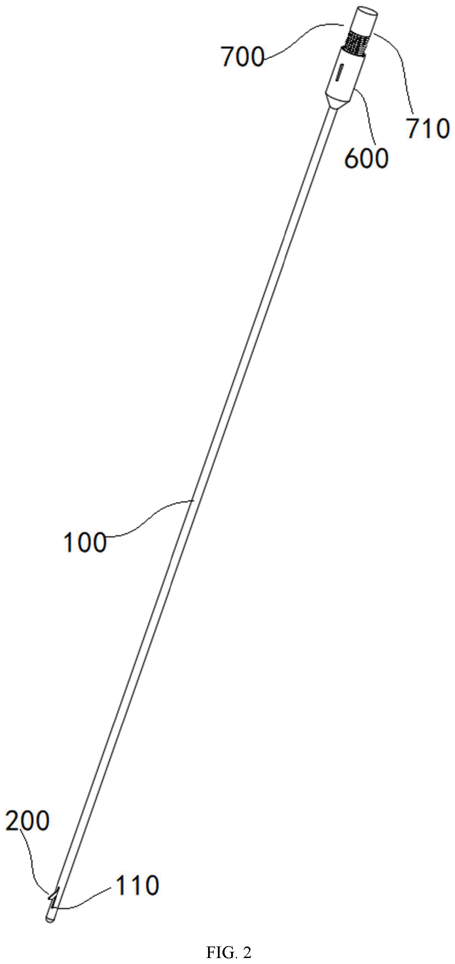

With reference to FIG. 1 to FIG. 17, the urethrotome for internal urethrotomy for the pet provided by this embodiment includes the urethrotome rod 100, the blade 200, and the guide rod 400; the blade slot 110 is formed in one side of the urethrotome rod 100, and a hollow structure 120 is arranged in the urethrotome rod 100 in a lengthwise direction and is in communication with the bottom of the blade slot 110; the blade 200 is rotatably accommodated in the blade slot 110 and has an accommodated state and a protruded state relative to the blade slot 100; one end of the guide rod 400 abuts against an edge of one end of the blade 200, and the other end of the guide rod 400 passes through the hollow structure 120 and forms the operation end at one end of the urethrotome rod 100, such that one end of the guide rod 400 is driven by the operation end to push the blade 200 to rotate and protrude from the opening of the blade slot 100; in this way, the blade 200 is switched from the accommodated state to the protruded; the urethrotome rod 100 can be of a slender hollow rod structure, and the guide rod 400 is movably inserted in the urethrotome rod 100; the guide rod 400 can be manually pulled to drive the pushing member 300 to push the blade 200 to upwarp, and thus the blade 200 can protrude from the opening of the blade slot 110; a cutting edge is formed in the side, facing the opening of the blade slot 110, of the blade 200, and thus the strictured and obstructed part of the urethra of the pet can be conveniently surgically cut; if the blade 200 is in the accommodated state, the urethrotome rod 100 can be more smoothly inserted into the strictured urethra of the pet; and if the blade 200 is in the protruded state, the strictured and obstructed part of the urethra can be conveniently cut with the blade 200.

As shown in FIG. 1 to FIG. 2 and FIG. 8 to FIG. 9, the urethrotome for internal urethrotomy for the pet provided by this embodiment includes the urethrotome rod 100, the blade 200, and the guide rod 400; the blade slot 110 is formed in one side of the urethrotome rod 100, and the hollow structure 120 is arranged in the urethrotome rod 100 in the lengthwise direction and is in communication with the bottom of the blade slot 110; the blade 200 is rotatably accommodated in the blade slot 110 and has the accommodated state and the protruded state relative to the blade slot 100; one end of the guide rod 400 abuts against the edge of one end of the blade 200, and the other end of the guide rod 400 passes through the hollow structure 120 and forms the operation end at one end of the urethrotome rod 100, such that one end of the guide rod 400 is driven by the operation end to push the blade 200 to rotate and protrude from the opening of the blade slot 100; in this way, the blade 200 is switched from the accommodated state to the protruded state; therefore, the blade 200 can be hidden and stored in the blade slot 110 during inserting the urethrotome into the urethra of the pet to prevent cutting the normal parts of the urethra of the pet, such that the urethrotome rod 100 can be inserted into the urethra of the pet more smoothly; and if the blade 200 reaches the strictured and obstructed part of the urethra of the pet, the guide rod 400 can be driven by means of the operation end to push the blade 200 to protrude from the opening of the blade slot 200 and perform surgical cutting on the strictured and obstructed part of the urethra of the pet; and the urethrotome has a simple and flexible structural design, and is more convenient to operate, the surgical operation difficulty is effectively reduced, and the surgical cutting efficiency is improved.

In one embodiment, as shown in FIG. 5 to FIG. 7 and FIG. 12 to FIG. 14, the urethrotome for internal urethrotomy for the pet further includes the pushing member 300 which is movably arranged in the hollow structure 120 of the urethrotome rod 100, and one end of the guide rod 400 is connected to the pushing member 300 and then abuts against the edge of one end of the blade 200 by the pushing member 300, such that the guide rod 400 can be driven by the operation end to drive the pushing member 300 to push the blade 200 to rotate and protrude from the opening of the blade slot 110.

In this embodiment, as shown in FIG. 5 to FIG. 7 and FIG. 12 to FIG. 14, a first end 230 of the blade 200 is pivotally connected to the urethrotome rod 100, a second end 240 of the blade 200 correspondingly abuts against the pushing member 300, such that the blade 200 can be pushed by the pushing member 300 to protrude from the opening of the blade slot 110 in a manner of rotating around a pivotal axis; because the urethra of the pet is quite strictured, in order to more smoothly insert the urethrotome for internal urethrotomy for the pet into the urethra of the pet, the urethrotome rod 100 is generally of the slender rod structure, the outer diameter of the slender rod structure is approximately 1-1.5 mm, the inner diameter of the hollow structure 120 is smaller than 1.0 mm, such that the space of the hollow structure 120 inside the urethrotome rod 100 is quite narrow; the inventor in test found that if some existing implementation modes utilizing large-sized medical cutters in other industries are adopted, one end of the guide rod 400 is directly rotatably connected to one end of the blade 200, because the pet urethra is quite strictured, the space of the hollow structure 120 of the urethrotome rod 100 is relatively narrow, consequently, the blade 200 is not provided with enough movement space to upwarp when rotatably connected to one end of the guide rod 400, or the height of the blade 200 upwarping from the blade slot 110 is relatively low, and basic requirements cannot be met, resulting in poor surgical cutting effects; in this embodiment, one end of the guide rod 400 separably abuts against the edge of one end of the blade 200, and the guide rod 400 is not directly rotatably connected to the blade 200, such that that the guide rod 400 can be slid by the operation end to move in the urethrotome rod 100 in the lengthwise position and to push the blade 200 to rotate and upwarp from the blade slot 110; in this way, the blade 200 is pushed to upwarp by abutting, the use effect is good, and it is easier to implemented in industrial manufacturing, simple in structural design, and low in machining process difficulty and production cost.

Specifically, as shown in FIG. 5 to FIG. 7 and FIG. 12 to FIG. 14, the blade slot 110 extends in the lengthwise position of the urethrotome rod 100, the first end 230 of the blade 200 is rotatably mounted in the blade slot 110 through a pivot, the pivotal axis of the pivot extends in the width direction of the blade slot 110, and the pivot can be a pin shaft or a metal wire; the pushing member 300 is movably inserted in the hollow structure 120 of the urethrotome rod 100, such that the guide rod 400 can be slid to drive the pushing member 300 to move in the urethrotome rod 100 in the lengthwise position and to push the blade 200 to protrude and upwarp from the opening of the blade slot 110 in a rotating mode.

Further, as shown in FIG. 5 to FIG. 7 and FIG. 12 to FIG. 14, the urethrotome for internal urethrotomy further includes a mounting body 500; a mounting groove 510 is formed in the side, facing the opening of the blade slot 110, of the mounting body 500 in the lengthwise position; the end, close to the blade 200, of the mounting groove 510 is open; in this way, the first end 230 of the blade 200 is rotatably arranged in the mounting groove 510 through the pivot, and the mounting body 500 is inserted in the hollow structure 120 of the urethrotome rod 100 so as to rotatably mount the blade 200 in the blade slot 110.

As shown in FIG. 5 to FIG. 7 and FIG. 12 to FIG. 14, the mounting body 500 is roughly cylindrical, mounting holes 520 are correspondingly formed in two sidewalls of the mounting groove 510 in the width direction, and a through hole 210 is formed in the first end 230 of the blade 200; during the assembling process, the first end 230 of the blade 200 can be inserted in the mounting groove 510 firstly, the pivot passes the mounting holes 520 in both sides of the mounting groove 510 and the through hole 210 in the blade 200 so as to rotatably mount the blade 200 on the mounting body 500; then the mounting body 500 and the blade 200 are inserted in the hollow structure 120 from the end of the urethrotome rod 100, and the blade 200 corresponds to the blade slot 110; preferably, the blade 200 is arranged adjacent to the other end of the blade slot 110 so that the blade 200 can be correspondingly assembled in the blade slot 110; specifically, a penetrating hole 530 is formed in the mounting body 500 in the lengthwise position so that the guide rod 400 can pass through the penetrating hole 530.

In this embodiment, as shown in FIG. 5 to FIG. 7 and FIG. 12 to FIG. 14, the second end 240 of the blade 200 is provided with a guide surface 220; the end, close to the blade 200, of the pushing member 300 is provided with a guide groove 310; the side, facing the opening of the blade slot 110, of the guide groove 310 is open; the second end 240 of the blade 200 is movably arranged in the guide groove 310 through the guide surface 220, such that the guide groove 310 is matched with the guide surface 220 to drive the pushing member 300 to push the blade 200 to protrude from the opening of the blade slot 110 in a manner of rotating around the pivot axis; specifically, during the assembling process, the pushing member 300 is connected to one end of the guide rod 400, then the pushing member 300 is inserted into the hollow structure 120 from the other end of the urethrotome rod 100, the guide groove 310 corresponds the guide surface 220 of the second end 240 of the blade 200, and finally the hollow structure 120 at the other end of the urethrotome rod 100 is covered with an end cap 130; specifically, the bottom of the blade slot 110 is in communication with the hollow structure 120, and the blade 200 is positioned between the pushing member 300 and the mounting body 500, as shown in FIG. 6 and FIG. 13; if the blade 200 is in the protruded state, the guide groove 310 in the pushing member 300 is matched with the mounting groove 510 in the mounting body 500 to clamp and fix the blade 200 and protrude and upwarp it from the opening of the blade slot 110.

As shown in FIG. 5 to FIG. 7 and FIG. 12 to FIG. 14, preferably, the guide surface 220 is an arc-shaped surface, a bottom wall surface of the guide groove 310 fits the guide surface 220, such that the guide groove 310 is matched with the guide surface 220 to transform the movement mode of the pushing member 300 into the rotating mode of the blade 200 more stably and reliably; and the structural design is compact, and the stability and reliability of the structural design are effectively improved.

In other embodiments, the pushing member 300 can be mounted in the mounting body 500 and mounted in the hollow structure 120 of the urethrotome rod 100 together with the mounting body 500; specifically, the mounting groove 510 is formed in the side, facing the opening of the blade slot 110, of the mounting body 500 in the lengthwise position; the other end of the blade 200 is rotatably arranged in the mounting groove 510 through the pivot; the penetrating hole 530 is formed in the mounting body 500 in the lengthwise direction and is in communication with the bottom of the mounting groove 510; the pushing member 300 is movably arranged in the penetrating hole 530; one end of the guide rod 400 is inserted into the penetrating hole 530 and connected to the pushing member 300, such that the mounting body 500 can be inserted into the hollow structure of the urethrotome rod 100 so as to rotatably mount the blade 200 in the blade slot 110; in the accommodated state, the blade 200 at least partially passes through the bottom of the mounting groove 510 and abuts against the pushing member 300 in the penetrating hole 530, and therefore the guide rod 400 can be driven by the pushing member 300 to push the blade 200 to rotate and protrude from the opening of the blade slot 110.

The mounting body 500 can be fixed to the inner wall of the hollow structure 120 of the urethrotome rod 100 by glue, or the urethrotome rod 100 is made of plastic materials, and therefore the inner wall of the urethrotome rod 100 can be shrunk by a hot melting process so as to be in tight fit with the mounting body 500 for fixation; it is simple and compact in structural design and convenient to produce and assemble, and the stability and reliability of structural design are effectively improved.

In this embodiment, as shown in FIG. 1 to FIG. 2 and FIG. 8 to FIG. 9, the urethrotome rod 100 is of a slender flexible rod structure, and the flexible rod structure is preferably made of PVC or PE flexible plastic materials; the guide rod 400 is made of metal materials and is inserted into the flexible rod structure to serve as a framework support, or the urethrotome rod 100 is of a sectional type, a flexible rod portion made of flexible plastic materials and a hard rod portion made of hard plastic or metal materials are combined to form the slender urethrotome rod 100, in which, the flexible rod structure or the flexible rod portion has a flexible and adaptable bending characteristic; and due to the flexible rod characteristic, the urethrotome rod 100 can be inserted into the urethra of the pet more smoothly, adapting to the bending of the urethra of the pet and avoiding injury that could be caused by using a straight and hard rod structure.

In this embodiment, as shown in FIG. 5 to FIG. 7 and FIG. 12 to FIG. 14, the urethrotome for internal urethrotomy further includes a handle 600 and a driving member 700; the handle 600 is arranged at one end of the urethrotome rod 100; the driving member 700 is movably arranged on the handle 600 and is in driving connection with the operation end of the guide rod 400, such that the guide rod 400 can be driven by the driving member 700 to drive the pushing member 300 to move in the lengthwise position of the urethrotome rod 100 and to push the blade 200 to protrude from the opening of the blade slot 110.

In order to facilitate operation, as shown in FIG. 3 to FIG. 7 and FIG. 10 to FIG. 14, in this embodiment, the handle 600 and the driving member 700 are arranged at one end of the urethrotome rod 100, the driving member 700 is movably arranged relative to the handle 600, such that the driving member 700 can be manually operated to move so as to drive the guide rod 400 to pull the pushing member 300 to push the blade 200 to protrude and upwarp from the opening of the blade slot 110; definitely, the driving member 700 can also be designed to be in an automatic mode, a driving motor is mounted on the handle 600, such that the guide rod 400 can be automatically driven by the driving motor to pull the pushing member 300 to push the blade 200 to protrude and upwarp from the opening of the blade slot 110.

In one embodiment, as shown in FIG. 3 to FIG. 7, the driving member 700 includes a rotating member 710; the rotating member 710 is in threaded connection with the handle 600 and is connected to the operation end of the guide rod 400; the pushing member 300 is provided with a threaded hole 320 in the lengthwise position; one end of the guide rod 400 is in threaded connection with the threaded hole 320, and the pushing member 300 is inserted into the hollow structure 120 of the urethrotome rod 100 in a non-rotatable mode, so that the rotating member 710 can be rotated to drive the guide rod 400 to move the pushing member 300.

As shown in FIG. 5 to FIG. 7, the rotating member 710 is sleeved with the handle 600 in a threaded connection mode; inner threads are formed in an inner cavity of the handle 600, outer threads are formed in the peripheral surface of the rotating member 710, and the rotating member 710 is inserted into the inner cavity of the handle 600 in the threaded connection mode; the operation end of the guide rod 400 passes through the inner cavity of the handle 600 and is fixedly connected to the rotating member 710; the pushing member 300 is roughly cylindrical; the threaded hole 320 is arranged in an eccentric position at the end, close to the blade 200, of the pushing member 300; and the outer threads are arranged in the peripheral surface of one end of the guide rod 400, thus the rotating member 710 can be conveniently rotated to drive the guide rod 400 to rotate; because the pushing member 300 is in threaded connection with one end of the guide rod 400 in an eccentric state, the pushing member 300 cannot be rotated in the hollow structure 120 of the urethrotome rod 100, and during rotating the rotating member 710 and the guide rod 400, the pushing member 300 can move in the hollow structure 120 in the lengthwise position of the urethrotome rod 100 and pushes the blade 200 to protrude and upwarp from the opening of the blade slot 110.

In another embodiment, as shown in FIG. 10 to FIG. 14, the driving member 700 includes a slider 720 which is connected to the operation end of the guide rod 400, at least one side of the slider 720 is provided with a sliding portion 721, the handle 600 is provided with a leading channel 611 in the lengthwise position, and the sliding portion 721 is arranged in the leading channel 611 in a sliding mode, such that the slider 720 can be manually slid to drive the guide rod 400 to move the pushing member 300 and to push the blade 200 to protrude and upwarp from the opening of the blade slot 110.

As shown in FIG. 12 to FIG. 14, two opposite sides of the slider 720 protrude outwards to form the sliding portions 721, the two opposite sides of the handle 600 extend towards the end away from the urethrotome rod 100 to form guide walls 610, the leading channels 611 are formed in the guide walls 610 in the lengthwise position, and the handle 600 and the guide walls 610 can be integrally molded and manufactured through elastic materials; during the assembling process, the two sliding portions 721 of the slider 720 are embedded into the leading channels 611 in the two guide walls 610 in a sliding mode respectively, and the operation end of the guide rod 400 passes through the inner cavity of the handle 600 and is fixedly connected to the slider 720; and in this way, the slider 720 can be manually slid to drive the guide rod 400 to move the pushing member 300 and to push the blade 200 to protrude and upwarp from opening of the blade slot 110.

In another embodiment, as shown in FIG. 10 to FIG. 14, the driving member 700 includes the rotating member 710 and the slider 720; the slider 720 is connected to the operation end of the guide rod 400, at least one side of the slider 720 is provided with the sliding portions 721, the handle 600 is provided with the leading channels 611 in the lengthwise position, the sliding portions 721 are arranged in the leading channels 611 in a sliding mode, and the rotating member 710 is in threaded connection with the handle 600 and is connected to the slider 720; and therefore, the rotating member 710 can be rotated to push the slider 720 to slide so as to drive the guide rod 400 to move the pushing member 300 and to push the blade 200 to protrude and upwarp from the opening of the blade slot 110.

As shown in FIG. 12 to FIG. 14, the two opposite sides of the slider 720 protrude outwards to form the sliding portions 721, the two opposite sides of the handle 600 extend towards the end away from the urethrotome rod 100 to form the guide walls 610, the leading channels 611 are formed in the guide walls 610 in the lengthwise position, and the handle 600 and the guide walls 610 can be integrally molded and manufactured through the elastic materials; during the assembling process, the two sliding portions 721 of the slider 720 are embedded into the leading channels 611 in the two guide walls 610 in the sliding mode respectively, the rotating member 710 is arranged between the handle 600 and the slider 720, and the rotating member 710 is sleeved with the handle 600 in the threaded connection mode; the inner threads are formed in the inner cavity of the handle 600, the outer threads are formed in the peripheral surface of the rotating member 710, and the rotating member 710 is inserted into the inner cavity of the handle 600 in the threaded connection mode; one end of the rotating member 710 abuts against one end of the slider 720, and the operation end of the guide rod 400 passes through the inner cavity of the handle 600 and the inner cavity of the rotating member 710 and is fixedly connected to the slider 720; in this way, during manually rotating the rotating member 710, the rotating member 710 can extend outwards relative to the handle 600 and pushes the slider 720 to slide away from the handle 600, such that the guide rod 400 can be driven to pull the pushing member 300 to move and to push the blade 200 to protrude and upwarp from the opening of the blade slot 110; the blade 200 is pushed to upwarp in a threaded rotation mode, thus the upwarping height of the blade 200 can be conveniently controlled and operated, and the stability and reliability of the structural design are improved.

In yet another embodiment, as shown in FIG. 15 to FIG. 17, this embodiment is different from the embodiments shown in FIG. 7 and FIG. 14; with reference to FIG. 17, in this embodiment, the pushing member 300 is omitted, and a slope 250 is arranged on the edge of one end of the blade 200; in the protruded state, the blade 200 abuts against one end of the guide rod 400 through the slope 250 so as to be supported for positioning; specifically, in the accommodated state, the blade 200 is accommodated in the blade slot 110, the lengthwise position of the blade 200 is parallel to a longitudinal axis of the urethrotome rod 100, and the slope 250 is inclined relative to the longitudinal axis of the urethrotome rod 100; if the blade 200 is in the protruded state, the other end of the blade 200 protrudes from the opening of the blade slot 110, the lengthwise position of the blade 200 is inclined relative to the longitudinal axis of the urethrotome rod 100, and the slope 250 is parallel to the longitudinal axis of the urethrotome rod 100, such that the blade 200 can abut against the peripheral wall of one side of the guide rod 400 through the slope 250 so as to be supported for positioning, thereby preventing the blade 200 from swinging during surgical cutting to affect the cutting effect.

Further, as shown in FIG. 15 to FIG. 17, the urethrotome for internal urethrotomy for the pet further includes the mounting body 500; the mounting groove 510 is formed in one side of the mounting body 500 and corresponds to the blade slot 110, the mounting holes 520 are formed in two opposite side walls of the mounting groove 510, and the through hole 210 is arranged in one end of the blade 200, such that the blade 200 is inserted into the mounting groove 510, and two ends of the pivot are inserted into the two mounting holes 520 respectively so as to realize rotatable mounting of the blade 200; in this way, the mounting body 500 is inserted into the hollow structure 120 of the urethrotome rod 100 so as to rotatably mount the blade 200 in the blade slot 110; the mounting body 500 is of a cylindrical rod structure; during assembling, the blade 200 is inserted into the mounting groove 510, then the pivot is inserted into the mounting holes 520 in the two opposite side walls of the mounting groove 510 and the through hole 210, then the mounting body 500 is inserted into the hollow structure 120 of the urethrotome rod 100, and the mounting groove 510 corresponds to the blade slot 110, therefore, the blade 200 can be rotatably mounted in the blade slot 110, and finally, the opening of the hollow structure 120 of the urethrotome rod 100 is covered with an end cap 130; the mounting body 500 can be fixed to the inner wall of the urethrotome rod 100 by glue, or the urethrotome rod 100 is made of the plastic materials, and the inner wall of the urethrotome rod 100 can by shrunk by the hot melting process so as to be in tight fit with the mounting body 500 for fixation; it is simple and compact in structural design and convenient to produce and assemble, and the stability and the reliability of the structural design are effectively improved.

In this embodiment, as shown in FIG. 15 to FIG. 17, the penetrating hole 530 is formed in the mounting body 500 in the lengthwise position and is in communication with the bottom of the mounting groove 510, and one end of the guide rod 400 is movably inserted into the penetrating hole 530; in the accommodated state, the blade 200 at least partially passes through the bottom of the mounting groove 510 and abuts against the guide rod 400 in the penetrating hole 530, such that the blade 200 can be pushed by the guide rod 400 to rotate and protrude from the opening of the blade slot 110; in this way, one end of the guide rod 400 can be driven by the operation end to push the blade 200 to rotate and protrude from the opening of the blade slot 110; moreover, the structure principles of the handles and driving members in the embodiments shown in FIG. 15 to FIG. 17 are basically the same as those of the handles and driving members in the embodiments shown in FIG. 7 and FIG. 14, the specific description and introduction of the handles and driving members in the above embodiments can be referred, and no more description will be made here.

In experimental test, the experiment was performed on pet cats to test the surgical cutting effect of the urethrotome for internal urethrotomy. The experiment was performed with four age groups across three breeds of pet cats, including a one-year group, a two-year group, a three-year group, and a four-year group, which were represented as a, b, c, and d respectively below. The three breeds of pet cats were Garfield, British Shorthair, and Ragdoll, represented as X, Y, and Z respectively. In total, there were 12 experimental groups, numbered as Xa, Xb, Xc, Xd, Ya, Yb, Yc, Yd, Za, Zb, Zc, and Zd, with ten cats in each group, totaling 120 pet cats. The urethrotome for internal urethrotomy in this embodiment was used for surgically cutting the urethras of the pet cats. After six months of postoperative observation, the final experimental results showed that all pet cats with urodialysis in each group were completely cured with no sequelae. After multiple experiments, physicians claimed that compared to the existing technology, this urethrotome for internal urethrotomy is very easy to use and operate, the surgical cutting procedure is relatively simple, and the cutting effect on the strictured or obstructed parts of the urethra of the pet is significantly better.

From the above description, it can be seen that the embodiments of the present invention have achieved the following technical effects:
the urethrotome for internal urethrotomy for the pet provided by this embodiment includes the urethrotome rod 100, the blade 200, and the guide rod 400; the blade slot 110 is formed in one side of the urethrotome rod 100, and the hollow structure 120 is arranged in the urethrotome rod 100 in the lengthwise direction and is in communication with the bottom of the blade slot 110; the blade 200 is rotatably accommodated in the blade slot 110 and has the accommodated state and the protruded state relative to the blade slot 100; one end of the guide rod 400 abuts against the edge of one end of the blade 200, and the other end of the guide rod 400 passes through the hollow structure 120 and forms the operation end at one end of the urethrotome rod 100, such that one end of the guide rod 400 is driven by the operation end to push the blade 200 to rotate and protrude from the opening of the blade slot 100; in this way, the blade 200 is switched from the accommodated state to the protruded state; therefore, the blade 200 can be hidden and stored in the blade slot 110 during inserting the urethrotome into the urethra of the pet to prevent cutting the normal parts of the urethra of the pet, such that the urethrotome rod 100 can be inserted into the urethra of the pet more smoothly; and if the blade 200 reaches the strictured and obstructed part of the urethra of the pet, the guide rod 400 can be driven by means of the operation end to push the blade 200 to protrude from the opening of the blade slot 200 and perform surgical cutting on the strictured and obstructed part of the urethra of the pet; and the urethrotome has a simple and flexible structural design, and is more convenient to operate, the surgical operation difficulty is effectively reduced, and the surgical cutting efficiency is improved.

It should be noted that the terms used here are only intended to describe specific embodiments and are not intended to limit the exemplary embodiments according to the present application. As used herein, unless otherwise explicitly stated in the context, the singular form is also intended to include the plural form. Additionally, it should be understood that when the terms "including" and/or "including" are used in this specification, they indicate the presence of features, steps, operations, devices, components, and/or combinations thereof.

Unless otherwise specified, the relative arrangement, numerical expressions, and values of the components and steps described in these embodiments do not limit the scope of the present application. At the same time, it should be understood that for ease of description, the dimensions of the various parts shown in the attached figure are not drawn according to the actual proportional relationship. For technologies, methods, and equipment known to ordinary technical personnel in related fields, detailed discussions may not be made, but in appropriate circumstances, such technologies, methods, and equipment should be considered as part of the authorization manual. In all the examples shown and discussed here, any specific value should be interpreted as merely illustrative and not restrictive. Therefore, other examples of exemplary embodiments may have different values. It should be noted that similar labels and letters represent similar items in the following figures. Therefore, once an item is defined in one figure, it does not need to be further discussed in subsequent figures.

In the description of this application, it should be understood that directional words such as "front, back, up, down, left, right", "horizontal, vertical, vertical, horizontal", and "top, bottom" usually indicate directional or positional relationships based on the directional or positional relationships shown in the accompanying drawings. This is only for the convenience of describing this application and simplifying the description. Unless otherwise stated, these directional words do not indicate or imply that the device or component referred to must have a specific orientation or be constructed and operated in a specific orientation, and therefore cannot be understood as limiting the scope of protection of this application; In addition, the directional words "inside" and "outside" refer to the inner and outer contours relative to each component itself.

It should also be noted that, unless otherwise specified and limited, terms such as "installation", "connection", "connection", "fixation", "setting" should be broadly understood, for example, they can be fixed connections, detachable connections, or integrated; It can be a mechanical connection or an electrical connection; It can be directly connected or indirectly connected through an intermediate medium, and it can be a connection within two components or an interaction relationship between two components. For ordinary technical personnel in this field, the specific meanings of the above terms in the present invention can be understood according to the specific situation.

In addition, if there are descriptions related to "first", "second", etc. in the embodiments of this application, the descriptions of "first", "second", etc. are only for descriptive purposes and cannot be understood as indicating or implying their relative importance or implying the number of technical features indicated. Therefore, the features that are limited to "first" and "second" can explicitly or implicitly include at least one of these features. In addition, the technical solutions between various embodiments can be combined with each other, but they must be based on the ability of ordinary technical personnel in this field to implement them. When the combination of technical solutions is contradictory or impossible to implement, it should be considered that this combination of technical solutions does not exist and is not within the scope of protection required by this application.

The above are the preferred embodiments of the present invention. It is to be noted that for those of ordinary skill in the art, various modifications and refinements can be made without departing from the principles of the present invention. Such modifications and refinements are also considered to be within the scope of the present invention.

## Claims

1. An urethrotome for internal urethrotomy for a pet, comprising:
an urethrotome rod, a blade slot being formed in one side of the urethrotome rod, and a hollow structure being arranged in the urethrotome rod in a lengthwise direction and being in communication with the bottom of the blade slot;
a blade which is rotatably accommodated in the blade slot and has an accommodated state and a protruded state relative to the blade slot; and
a guide rod of which one end abuts against an edge of one end of the blade while the other end passes through the hollow structure and forms an operation end at one end of the urethrotome rod, one end of the guide rod being driven by the operation end to push the blade to rotate and protrude from an opening of the blade slot, so as to switch the blade from the accommodated state to the protruded state.

2. The urethrotome for internal urethrotomy for the pet according to claim 1, wherein a slope is arranged on the edge of one end of the blade; and in the protruded state, the blade abuts against one end of the guide rod through the slope so as to be supported for positioning.

3. The urethrotome for internal urethrotomy for the pet according to claim 2, wherein in the accommodated state, the blade is accommodated in the blade slot, the lengthwise position of the blade is parallel to a longitudinal axis of the urethrotome rod, and the slope is inclined relative to the longitudinal axis of the urethrotome rod; and in the protruded state, the other end of the blade protrudes from the opening of the blade slot, the lengthwise position of the blade is inclined relative to the longitudinal axis of the urethrotome rod, and the slope is parallel to the longitudinal axis of the urethrotome rod.

4. The urethrotome for internal urethrotomy for the pet according to claim 1, wherein the blade slot extends in the lengthwise position of the urethrotome rod, one end of the blade is rotatably mounted in the blade slot through a pivot, and the pivot extends in a width direction of the blade slot.

5. The urethrotome for internal urethrotomy for the pet according to claim 4, further comprising a mounting body, wherein a mounting groove is formed in one side of the mounting body and corresponds to the blade slot, mounting holes are formed in two opposite side walls of the mounting groove, the blade is inserted into the mounting groove, and two ends of the pivot are inserted into the two mounting holes respectively so as to realize rotatable mounting the blade; and the mounting body is inserted into the hollow structure of the urethrotome rod so as to rotatably mount the blade in the blade slot.

6. The urethrotome for internal urethrotomy for the pet according to claim 5, wherein a penetrating hole is formed in the mounting body in the lengthwise direction and is in communication with the bottom of the mounting groove; one end of the guide rod is movably inserted into the penetrating hole; in the accommodated state, the blade at least partially passes through the bottom of the mounting groove and abuts against the guide rod in the penetrating hole, and then the blade can be pushed by the guide rod to rotate and protrude from the opening of the blade slot.

7. The urethrotome for internal urethrotomy for the pet according to claim 1, further comprising a pushing member, wherein the pushing member is movably arranged in the hollow structure of the urethrotome rod, and one end of the guide rod is connected to the pushing member and then abuts against the edge of one end of the blade by the pushing member; and the guide rod can be driven by the operation end to drive the pushing member to push the blade to rotate and protrude from the opening of the blade slot.

8. The urethrotome for internal urethrotomy for the pet according to claim 7, wherein the blade slot extends in the lengthwise position of the urethrotome rod, the other end of the blade is rotatably mounted in the blade slot through the pivot, and a pivotal axis of the pivot extends in the width direction of the blade slot.

9. The urethrotome for internal urethrotomy for the pet according to claim 8, further comprising the mounting body, wherein the mounting groove is formed in the side, facing the opening of the blade slot, of the mounting body in the lengthwise position, the other end of the blade is rotatably inserted in the mounting groove through the pivot, and the mounting body is inserted in the hollow structure of the urethrotome rod so as to rotatably mount the blade in the blade slot.

10. The urethrotome for internal urethrotomy for the pet according to claim 7, wherein a guide surface is arranged on the edge of one end of the blade; a guide groove is formed in the end, close to the blade, of the pushing member; one end of the blade is movably arranged in the guide groove through the guide surface; and the pushing member abuts against the guide surface through the guide groove to push the blade to rotate and protrude from the opening of the blade slot.

11. The urethrotome for internal urethrotomy for the pet according to claim 8, further comprising the mounting body, wherein the mounting groove is formed in the side, facing the opening of the blade slot, of the mounting body in the lengthwise position; the other end of the blade is rotatably arranged in the mounting groove by the pivot; the penetrating hole is formed in the mounting body in the lengthwise direction and is in communication with the bottom of the mounting groove; the pushing member is movably arranged in the penetrating hole; one end of the guide rod is inserted into the penetrating hole and connected to the pushing member, and the mounting body is inserted into the hollow structure of the urethrotome rod so as to rotatably mount the blade in the blade slot; and in the accommodated state, the blade at least partially passes through the bottom of the mounting groove and abuts against the pushing member in the penetrating hole, and then the guide rod can be driven by the pushing member to push the blade to rotate and protrude from the opening of the blade slot.

12. The urethrotome for internal urethrotomy for the pet according to claim 1, wherein the urethrotome rod is of a flexible rod structure, or the urethrotome rod consists of a flexible rod portion and a hard rod portion.

13. The urethrotome for internal urethrotomy for the pet according to claim 1, further comprising a handle and a driving member, wherein the handle is arranged at one end of the urethrotome rod, the driving member is movably arranged on the handle and is in driving connection with the operation end of the guide rod, and the guide rod is driven by the driving member to push the blade to protrude from the opening of the blade slot.

14. The urethrotome for internal urethrotomy for the pet according to claim 13, wherein the driving member comprises a rotating member, the rotating member is in threaded connection with the handle and is connected to the operation end of the guide rod, and the rotating member can be rotated to drive the guide rod to push the blade to rotate and protrude from the opening of the blade slot; or
the driving member comprises a slider which is connected to the operation end of the guide rod, at least one side of the slider is provided with a sliding portion, the handle is provided with a leading channel in the lengthwise position, and the sliding portion is arranged in the leading channel in a sliding mode; and the slider can be slid to drive the guide rod to push the blade to rotate and protrude from the opening of the blade slot.

15. The urethrotome for internal urethrotomy for the pet according to claim 7, further comprising the handle and the driving member, wherein the handle is arranged at one end of the urethrotome rod; the driving member comprises the rotating member, the rotating member is in threaded connection with the handle and is connected to the operation end of the guide rod; the pushing member is provided with a threaded hole in the lengthwise position, and one end of the guide rod is in threaded connection with the threaded hole; the pushing member is inserted into the hollow structure of the urethrotome rod in a non-rotatable mode, and the rotating member can be rotated to drive the guide rod to move the pushing member; or
further comprising the handle and the driving member, wherein the handle is arranged at one end of the urethrotome rod; the driving member comprises the slider which is connected to the operation end of the guide rod; the sliding portion is arranged on at least one side of the slider; the handle is provided with the leading channel in the lengthwise position, and the sliding portion is arranged in the leading channel in a sliding mode; the slider can be slid to drive the guide rod to move the pushing member; or
further comprising the handle and the driving member, wherein the handle is arranged at one end of the urethrotome rod; the driving member comprises the rotating member and the slider; the slider is connected to the operation end of the guide rod, and the sliding portion is arranged on at least one side of the slider; the handle is provided with the leading channel in the lengthwise position, and the sliding portion is arranged in the leading channel in a sliding mode; the rotating member is in threaded connection with the handle and abuts against the slider; and the rotating member can be rotated to push the slider to slide so as to drive the guide rod to move the pushing member.
